# EUROPEAN PATENT SPECIFICATION

(11) **EP 2 260 768 B1**
(45) Date of publication and mention of the grant of the patent: **16.03.2016**
(21) Application number: 09728097.8
(22) Date of filing: 24.03.2009
(51) Int. Cl.: A61B 17/12

(54) **CLOSING DEVICE FOR MEDICAL USE**
VERSCHLUSSVORRICHTUNG ZUR MEDIZINISCHEN VERWENDUNG
DISPOSITIF D'OBTURATION À USAGE MÉDICAL

(30) Priority: 31.03.2008 JP 2008093548
(43) Date of publication of application: 15.12.2010
(73) Proprietor: Terumo Kabushiki Kaisha, Tokyo 151-0072 (JP)
(72) Inventor: ANZAI, Takao, Ashigarakamigun Kanagawa 259-0151 (JP)
(74) Representative: Dossmann, Gérard
(86) International application number: PCT/JP2009/055857
(87) International publication number: WO 2009/122971

(56) References cited:
- JP-A- 2001 079 011
- JP-B2- 61 020 304
- JP-T- 2003 503 162
- JP-T- 2003 511 188
- JP-T- 2005 537 830
- US-A1- 2001 046 518
- US-A1- 2004 060 563
- US-A1- 2004 098 028
- US-A1- 2006 155 303
- US-A1- 2007 078 480

## Description

### BACKGROUND OF THE INVENTION

### Field of the Invention

The present invention relates to a medical blocking tool which is placed inside of a lumen of a body to block the lumen, and in particular a medical blocking tool according to the preamble of claim 1, such as it is known for example from US 2004/098028 A1.

### Description of the Related Art

Emphysema is a lesion which is formed in the main by the inhalation of harmful substances generated by smoking or the like, and causes a wide range of damage to the alveoli and distal airways of the lungs. The formation of the lesion is chronically progressive, and the respiratory function of a patient having such a chronic progressive lesion is remarkably inhibited.

The main therapies for a patient having such emphysema are largely classified into medical therapy and surgical therapy. However, in medical therapy, it is possible to relive the patient's symptomatic state, but it is difficult to stop the progression of the emphysema.

Further, examples of surgical therapy include lung volume reduction surgery and lung transplant, but all of them have problems in that the patient needs to undergo a major procedure, the burden on the patient is extraordinarily large, and a huge amount of money is spent. For these reasons, surgical therapies cannot be easily carried out.

Therefore, as a medical blocking tool capable of solving the problems with the surgical therapies for emphysema and used in therapy for the emphysema, JP-A-2004-24864 discloses a resinous bronchial tube blocking material (blocking material) which is formed in a conical trapezoid shape. The blocking material is placed in a predetermined portion of a bronchial tube by the use of a bronchoscope (an endoscope for a bronchial tube) or the like to block the predetermined portion of the bronchial tube, where the outer diameter of the blocking material is set to be larger than the inner diameter of the portion (blocking target portion) to be blocked in the bronchial tube.

Since the outer diameter of the blocking material is larger than the inner diameter of the lumen of the bronchoscope, the blocking material cannot be inserted into the lumen of the bronchoscope. For this reason, the blocking material is gripped at the front end side of the bronchoscope by a clamp or the like inserted through the lumen of the bronchoscope and protruding outward from the front end of the bronchoscope, and is transported and placed at the blocking target portion of the bronchial tube together with the bronchoscope.

However, since the outer diameter of the blocking material of the related art is larger than the inner diameter of the blocking target portion of the bronchial tube, and the blocking material is gripped at the front end side of the bronchoscope by the use of the clamp to be transported and placed to the blocking target portion, many years of experience and high skill levels are required to perform the treatment.

In addition, the blocking material is generally placed at each of a plurality of positions, but whenever the placement for each blocking material is completed, it is necessary to perform a series of operations whereby the bronchoscope is extracted from inside the body, the subsequent blocking material to be placed is gripped by the clamp protruding outward from the front end of the bronchoscope, and then the bronchoscope is re-inserted into the body. For this reason, the treatment time is lengthy, the burden on the patient is exceptionally large.

### SUMMARY OF THE INVENTION

An object of the invention is to provide a medical blocking tool which can be quickly, easily, and reliably placed inside of a lumen of a body.

In order to achieve the above-described object, there is provided a medical blocking tool according to claim 1.

The dependent claims relate to advantageous embodiments. In the medical blocking tool with the above-described configuration a fixation assisting means is provided to assist fixation when the swelling section is fixed to the inside of the lumen of the body. Accordingly, it is possible to more reliably fix the medical blocking tool to the inside of the lumen of the body.

With such a configuration, the medical blocking tool is transported to the portion (blocking target portion) to be blocked in the lumen of the body while the swelling section is in its contracted state (the state before the swelling operation), and is fixed (placed) thereto while the swelling section is swelled (expanded). Accordingly, it is possible to easily and promptly place the medical blocking tool.

In addition, since the medical blocking tool is fixed to the inside of the lumen of the body to block the lumen when the swelling section is swelled, it is possible to reliably fix the medical blocking tool, and to reliably block the lumen of the body.

For example, even when the surface of the body tissue defining the lumen of the body is uneven, since the medical blocking tool can reliably follow the shape of the unevenness, the medical blocking tool can be reliably adhered to the surface of the body tissue.

Further, since the physical irritation of the swelling section with respect to the body tissue is small, it is possible to suppress granulation or the like.

Furthermore, since the swelling section of the medical blocking tool is in a contracted state before the medial blocking tool is placed inside of the lumen of the body, for example, when the lumen of the device (for example, a bronchoscope or the like when the medical blocking tool is placed inside of the bronchial tube) used to place the medical blocking tool is used, it is possible to transport the medical blocking tool to the front end of the device. Accordingly, it is possible to place the medical blocking tool at a plurality of positions while the medical blocking tool is inserted inside the body. Therefore, since it is possible to shorten the treatment time, it is possible to reduce the burden on the patient.

In the medical blocking tool with the above-described configuration, the swelling material is preferably a gel polymer.

Accordingly, the swelling material can be selectively swelled (expanded) or contracted in response to the characteristics of the contacting liquid.

In the medical blocking tool with the above-described configuration, a part of a molecular structure of the gel polymer preferably has an anionic group.

Accordingly, the swelling rate (expansion rate) and the contraction rate of the gel polymer are selectively increased in accordance with the concentration of contained ions, composition of contacting liquid or the like. In addition, the swelling rate (expansion rate) and the contraction rate of the environmentally-sensitive gel polymer are selectively increased in accordance with the concentration of contained ions, composition of contacting liquid or the like. Further, since the hydrophilic properties of the environmentally-sensitive gel polymer are increased, the gel polymer can be swelled by actively absorbing a larger amount of liquid. Furthermore, since the hydrophilic properties of the gel polymer are increased, the gel polymer can be swelled by actively absorbing a larger amount of liquid.

In the medical blocking tool with the above-described configuration, it is preferred that the volume of the gel polymer is increased when contacting a liquid deprotonating the anionic group, or the volume thereof is decreased when contacting a liquid protonating the anionic group.

Accordingly, the gel polymer is swelled when contacting a liquid exhibiting deprotonation, and is contracted when contacting a liquid exhibiting protonation.

In the medical blocking tool with the above-described configuration, it is preferred that the gel polymer contains at least one type of monomer component selected from acrylic acid, methacrylic acid, and their derivatives.

Accordingly, since the gel polymer containing them has an anionic group, its environmental sensitivity is further improved.

In the medical blocking tool with the above-described configuration, the gel polymer preferably contains an ethylenically unsaturated compound as a cross-linking agent.

Accordingly, it is possible to improve the mechanical characteristics of the gel polymer.

In the medical blocking tool with the above-described configuration, the core section may be formed as a bar-shaped wire rod, and the swelling section is preferably formed along the wire rod to coat the wire rod.

Accordingly, it is possible to quickly, easily, and reliably place the medical blocking tool inside of the lumen of the body.

In the medical blocking tool with the above-described configuration, the core section may be formed as a coil-shaped wire rod, and the swelling section is preferably formed as a coil shape along the wire rod to coat the wire rod.

Accordingly, when the swelling section is swelled, a gap in the swelling section is removed, so that the inside of the lumen of the body is blocked.

In the medical blocking tool with the above-described configuration, the core section may be formed as a coil-shaped wire rod, and the swelling section is preferably formed as a bar shape to cover the entire portion of the wire rod.

Accordingly, the contact area between the core section and the swelling section is increased, and hence the adhesion between the core section and the swelling section is improved.

In the medical blocking tool with the above-described configuration, the core section preferably has a contrast property.

Accordingly, it is possible to allow the core section (the medical blocking tool) to have X-ray contrast property, and to position the medical blocking tool inside of the bronchial tube while checking (watching) the position of the medical blocking tool using X-ray fluoroscopy.

In the medical blocking tool with the above-described configuration, a surface of the core section is preferably subjected to a roughing process.

Accordingly, the contact area with respect to the swelling section is increased, and hence adhesion with respect to the swelling section is improved.

In the medical blocking tool with the above-described configuration, the medical blocking tool is preferably used to block a bronchial tube.

In the medical blocking tool with the above-described configuration, it is preferable that the fixation assisting means is formed of an elastic material, and the fixation assisting means is contracted when applying an external force against its elasticity, and is expanded when releasing the external force.

Accordingly, it is possible to more reliably fix the medical blocking tool to the inside of the lumen of the body.

Therefore, it is possible to quickly, easily, and reliably place the medical blocking tool inside of the bronchial tube.

### BRIEF DESCRIPTION OF THE DRAWINGS

Fig. 1 is a perspective view showing a first illustrative example of a medical blocking tool not falling under the scope of the invention.
Fig. 2 is a longitudinal sectional view showing a medical blocking tool shown in Fig. 1.
Fig. 3 is a diagram illustrating instructions for use of the medical blocking tool shown in Fig. 1.
Fig. 4 is a diagram illustrating instructions for use of the medical blocking tool shown in Fig. 1.
Fig. 5 is a diagram illustrating instructions for use of the medical blocking tool shown in Fig. 1.
Fig. 6 is a side view showing a second illustrative example of a medical blocking tool not falling under the scope of the invention.
Fig. 7 is a side view showing a third illustrative example of a medical blocking tool not falling under the scope of the invention.
Figs. 8A, 8B, and 8C are longitudinal sectional views (diagrams illustrating instructions for use) showing a preferred embodiment of the medical blocking tool of the invention.

### DESCRIPTION OF PREFERRED EMBODIMENT

Hereinafter, a medical blocking tool of the invention will be described in detail with reference to the preferred embodiments shown in the accompanying drawings.

In addition, the invention may be applied to various medical blocking tools which are placed inside a lumen of a body (to be fixed to body tissue defining the lumen of the body) and close the lumen of the body. However, in the embodiments to be described below, a case will be representatively described in which the invention is applied to a bronchial tube blocking tool (a bronchial tube closing tool) used to block (close) a bronchial tube.

### First illustrative example

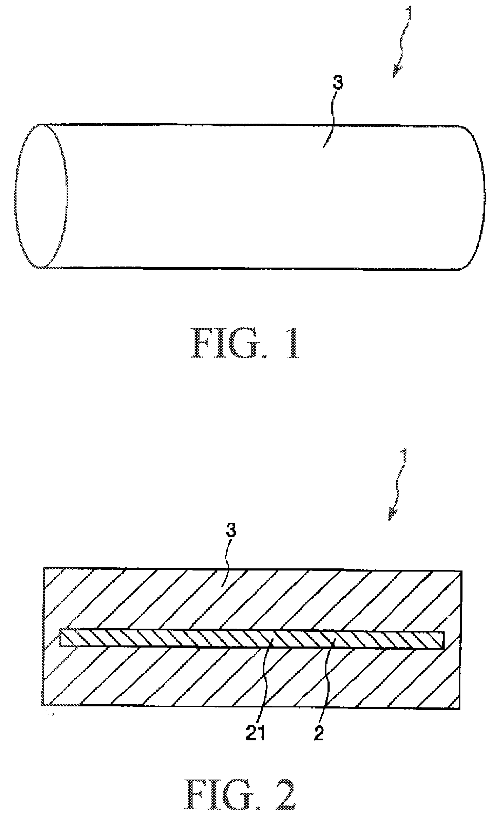

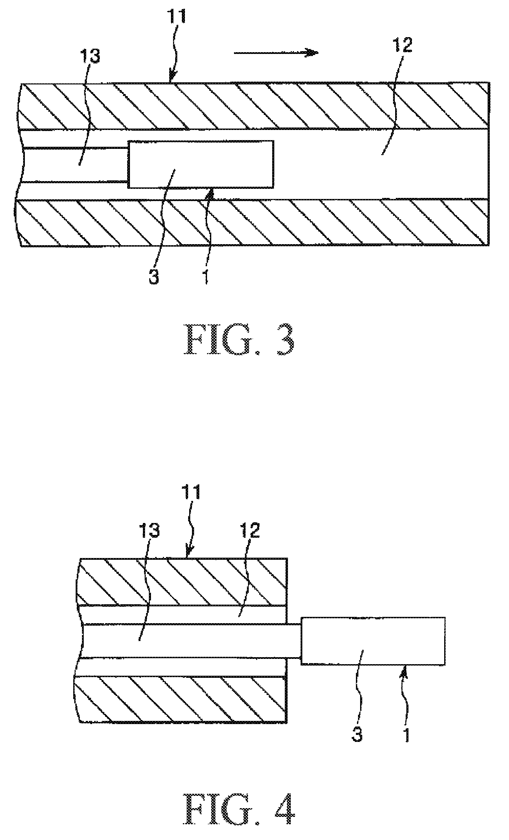

Fig. 1 is a perspective view showing a first illustrative example of a medical blocking tool not falling under the scope of the claims. Fig. 2 is a longitudinal sectional view showing a medical blocking tool shown in Fig. 1. Figs. 3 to 5 are diagrams illustrating instructions for use of the medical blocking tool shown in Fig. 1.

In addition, in the description below, the right of Figs. 3 and 4 indicates the "front end", and the left thereof indicates the "base end (rear end)".

The medical blocking tool 1 shown in the drawings is a tool which is fixed (placed) inside of the bronchial tube to block the bronchial tube.

The medical blocking tool 1 includes a core section 2 and a swelling section 3 provided (fixed) around the core section 2.

The core section 2 has the function of a reinforcing member for reinforcing the swelling section 3 (the medical blocking tool 1). The core section 2 is formed as a bar-shaped wire rod 21, and is formed in a linear shape in this embodiment. In addition, the core section 2 may have a solid shape, a hollow shape, or a cylindrical shape.

It is desirable that the surface of the core section 2 is subjected to a roughing process. Accordingly, the contact area with respect to the swelling section 3 is increased, and hence the adhesion with respect to the swelling section 3 is improved.

It is desirable that the core section 2 has a contrast property with respect to, for example, X-ray fluoroscopy (including CT scan), MRI, or the like. In addition, it is desirable that the core section 2 is formed of metal materials. Examples of metal materials forming the core section 2 include, for example, stainless steel, a hyperelastic alloy, a cobalt-based alloy, or a precious metal such as gold, platinum, and tungsten, or an alloy containing them (for example, platinum-iridium alloy). Particularly, when the core section 2 is formed of a radiopaque material such as a precious metal, that is, a material having a contrast property with respect to X-rays, it is desirable in that the contrast property with respect to the X-rays is obtained in the core section 2 (the medical blocking tool 1), and the medical blocking tool 1 can be placed inside the bronchial tube while checking (watching) the position of the medical blocking tool 1 under the X-ray fluoroscopy.

The dimensions (the length, the outer diameter, and the like) of the core section 2 are not particularly limited, and are appropriately determined in accordance with the portion (position) or case of placing the medical blocking tool 1. Generally, it is desirable that the length of the core section 2 is about 5 to 100 mm, and more desirable that the length is about 10 to 50 mm. In addition, it is desirable that the outer diameter of the core section 2 is about 0.01 to 1 mm, and more desirable that the outer diameter is about 0.02 to 0.5 mm.

The swelling section 3 is formed of a swelling material which is swelled (expanded) by absorbing a liquid so that the volume thereof is increased. The swelling section 3 is adapted to be swelled until the bronchial tube is blocked while coming into close contact with the inner wall of the bronchial tube in the portion to be blocked (blocking target portion) in the bronchial tube. That is, the medical blocking tool 1 is fixed to the inside of the bronchial tube in accordance with the swelling operation of the swelling section 3 to block the bronchial tube. In the following description, the description is made on the assumption that the swelling section 3 is in a contracted state (a state before a swelling operation) unless there is no particular remark.

The swelling section 3 is provided along the wire rod 21 of the core section 2, and coats the entire part of the wire rod 21 (core section 2). That is, the core section 2 is buried in the swelling section 3. In addition, in this embodiment, the swelling section 3 is formed in a bar shape, and the medical blocking tool 1 is formed in a cylindrical shape.

The dimensions (the length, the outer diameter, and the like) of the swelling section 3 (medical blocking tool 1) are not particularly limited, and are appropriately determined in accordance with the portion (position) or case of placing the medical blocking tool 1. Generally, it is desirable that the length of the swelling section 3 (medical blocking tool 1) is about 5 to 100 mm, and more desirable that the length is about 10 to 50 mm.

In addition, the outer diameter of the swelling section 3 (medical blocking tool 1) is set to be smaller than the inner diameter of a lumen of a bronchoscope to be described later. Accordingly, the medical blocking tool 1 is movable in the inside of the lumen of the bronchoscope. In addition, the outer diameter of the swelling section 3 (medical blocking tool 1) is set to a degree that the bronchial tube can be blocked when the swelling section 3 is swelled. Specifically, it is desirable that the outer diameter of the swelling section 3 (medical blocking tool 1) is about 1 to 50 mm, and more desirable that the outer diameter is about 5 to 30 mm.

The swelling material forming the swelling section 3 is not particularly limited so long as the swelling material is swelled by absorbing a liquid so that the volume thereof is increased, but a gel polymer is desirable. Further, in this embodiment, a case will be described in which gel polymer is used as the swelling material.

The gel polymer (polymer) forming the swelling section 3 has a property in which the volume is changed in response to contact with a liquid.

That is, the gel polymer in its dry state (maximally contracted state) before gelation is not in a gelation state, but is swelled (expanded) to enter a gelation state when the inside thereof absorbs a liquid. In addition, the gel polymer swelled in this way discharges the liquid absorbed thereinto to enter a contracted state when the gel polymer comes into contact with a liquid having a predetermined property.

That is, the gel polymer can be selectively swelled (expanded) or contracted in response to the properties of the contacting liquid. Such a gel polymer is generally referred to as "environmentally-sensitive gel polymer".

The environmentally-sensitive gel polymer is mainly used as a cross-linker which is formed by polymerizing and linking environmentally-sensitive monomer components or pre-polymer components, and is composed of a polymer having a three-dimensional mesh structure.

Such an environmentally-sensitive gel polymer has void holes in gaps of molecular chains. The void holes are expanded and contracted in response to a binding force between the molecular chains, but the binding force between the molecular chains are changed depending on the environment where the gel polymer exists. Due to such a property, the environmentally-sensitive gel polymer can absorb a liquid into the void holes, or discharge a liquid absorbed in the void holes in response to the change of the environment.

Examples of the monomer component or the pre-polymer component of the environmentally-sensitive gel polymer include acrylic acid, methacrylic acid, or derivatives thereof, styrenesulfonic acid, vinyl sulfonic acid, vinyl phosphoric acid, and one type of the above or mixture of two or more types of the above can be used.

Among them, particularly, acrylic acid, methacrylic acid, or such derivative is desirably used. Acrylic acid, methacrylic acid, or such derivative is the environmentally-sensitive monomer component (pre-polymer component), and the gel polymer containing them has an ionic functional group to be described later. Accordingly, the environmental sensitivity of the gel polymer is increased.

In addition, it is desirable that the monomer component or the pre-polymer component of the environmentally-sensitive gel polymer contains ethylenically unsaturated monomer such as 2-hydroxyethylacrylate, 2-hydroxyethylmethacrylate, acrylic amide, or methacrylic amide, or such derivative, in addition to the above-described examples.

Among them, particularly, acrylic amide is desirably used. By containing the acrylic amide, it is possible to improve the mechanical characteristics of the gel polymer.

In addition, a part of the molecular structure of the monomer component or the pre-polymer component has an ionic functional group. Accordingly, the swelling rate (expansion rate) and the contraction rate of the environmentally-sensitive gel polymer are selectively increased in accordance with the concentration of contained ions, composition of contacting liquid or the like. In addition, since the hydrophilic properties of the environmentally-sensitive gel polymer are increased, the gel polymer can be swelled by actively absorbing a larger amount of liquid.

The ionic functional group is an anionic group, and the environmentally-sensitive gel polymer containing such an ionic functional group is swelled by deprotonation, so that the volume thereof is increased. In addition, the swelled environmentally-sensitive gel polymer is contracted by protonation, so that the volume thereof is decreased. Accordingly, the environmentally-sensitive gel polymer containing the anionic group is swelled when contacting a liquid exhibiting deprotonation, and then is contracted when contacting a liquid exhibiting protonation.

That is, in its dry state (maximally contracted state) before gelation, the volume of the environmentally-sensitive gel polymer containing the anionic group is at its smallest. On the other hand, in the gelation state contacting a liquid, the swelling rate becomes larger and the volume increases as the pH of the contacting liquid becomes larger. Likewise, the volume of the environmentally-sensitive gel polymer containing the anionic group has a positive correlation with respect to the pH of the liquid contacting the gel polymer.

Examples of the anionic group include a carboxylic acid group, a mercapto group, a phosphoric acid group, a sulfonic acid group, and the like.

In addition, the ionic functional group may be a cationic group. In this case, the environmentally-sensitive gel polymer is swelled by protonation, so that the volume thereof is increased. In addition, the swelled environmentally-sensitive gel polymer is contracted by deprotonation, so that the volume thereof is decreased. Accordingly, the environmentally-sensitive gel polymer containing the cationic group is swelled when contacting a liquid exhibiting protonation, and then is contracted when contacting a liquid exhibiting deprotonation.

That is, in the dry state (maximally contracted state) before gelation, the volume of the environmentally-sensitive gel polymer containing the cationic group is at its smallest. On the other hand, in the gelation state contacting a liquid, the swelling rate becomes larger and the volume increases as the pH of the contacting liquid decreases. Likewise, the volume of the environmentally-sensitive gel polymer containing the cationic group has a negative correlation with respect to the pH of the liquid contacting the gel polymer.

Examples of the cationic group include an amino group, an ammonium group, and the like.

However, in the description of the instructions for use of the medical blocking tool 1 to be described later, a case in which the ionic functional group is the anionic group will be representatively described.

In addition, in the invention, the swelling material forming the swelling section 3 is not limited to the gel polymer. For example, a material may be used which is swelled by absorbing a liquid thereinto, but does not enter a gelation state.

Next, a method of manufacturing the medical blocking tool 1 will be described.
[1] First, the raw material for forming the swelling section 3 is prepared.
   A monomer component (pre-polymer component) as a raw material of the gel polymer, a cross-linking agent, a polymerization initiator, and a solvent are prepared. Then, a solution is prepared by mixing them. Accordingly, the monomer component is polymerized and linked stereoscopically, thereby obtaining a gel polymer having a three-dimensional mesh structure.
   In addition, it is desirable that the content ratio of the environmentally-sensitive monomer component among the total monomer components is about 10 to 50 mass%, and more desirable that the content ratio is about 10 to 30 mass%.
   Further, the content ratio of the monomer component in the solution is not particularly limited, but is desirably about 20 to 30 mass%.
   Examples of the cross-linking agent include ethylenically unsaturated compounds such as N,N'-methylenebisacrylamide, ethylene glycol di(meth)acrylate, diethylene glycol di(meth)acrylate, triethylene glycol di(meth)acrylate, polyethylene glycol di(meth)acrylate, trimethylolpropane tri(meth)acrylate, pentaerythritol tri(meth)acrylate, divinylbenzene, and divinylether. Since the ethylenically unsaturated compound functions as a cross-linking agent together with the monomer component to reliably form a three-dimensional mesh structure, the ethylenically unsaturated compound is particularly suitable as the cross-linking agent for forming the swelling section 3.
   Among them, the N, N'-methylenebisacrylamide is more desirably used.
   The content ratio of the cross-linking agent among the solution is not particularly limited. However, it is desirable that the content ratio is less than about 1 mass%, and more desirable that the content ratio is less than 0.1 mass%.
   In addition, examples of the polymerization initiator include ammonium persulfate, N, N, N', N'-tetramethylethylene diamine, and the like.
   Further, examples of the solvent include water, ethanol, and the like.
   Furthermore, if necessary, a pore-forming agent may be contained in the solution. Accordingly, the swelling section 3 can have a porous structure. Since the swelling section 3 with such a porous structure has a large surface area, the liquid absorption speed is high, and the swelling speed (expansion speed) is high.
   Examples of the pore-forming agent include sodium chloride, potassium chloride, ice, saccharose, sodium bicarbonate, and the like.
   In addition, it is desirable that the average particle diameter of the pore-forming agent is about 1 to 25 µm, and more desirable that the average particle diameter is about 3 to 10 µm.
   Further, it is desirable that the content ratio of the pore-forming agent in the solution is about 5 to 50 mass%, and more desirable that the content ratio is about 10 to 20 mass%.
   Furthermore, if necessary, particles formed of a material opaque to X-rays may be contained in the solution. Accordingly, the swelling section 3 can obtain the X-ray contrast property, and the position of the swelling section 3 (the medical blocking tool 1) can be easily checked in the X-ray fluoroscopy.
[2] Subsequently, the obtained solution is coated on the core section 2, so that a gel polymer layer, that is, the swelling section 3 is formed on the surface of the core section 2. Accordingly, it is possible to obtain the swelling section 3 which is in a swelled state.
[3] Subsequently, the swelling section 3 is cleaned by a cleaning liquid. Accordingly, unreacted residual monomer components, the pore-forming agent, and the like are removed.
[4] Subsequently, the swelling section 3 which is in the swelled state is dried. Accordingly, the swelling section 3 which is in a dry state (maximally contracted state) is formed, thereby obtaining the medical blocking tool 1.

Next, an example of the instructions for use (operations) of the medical blocking tool 1, that is, the procedure of placing the medical blocking tool 1 inside of the bronchial tube will be described.

As shown in Fig. 3, the medical blocking tool 1 is separably connected and held to the front end of an operation wire 13 as a pressure member. For example, the connection and the separation (separation of the medical blocking tool 1) between the medical blocking tool 1 and the operation wire 13 can be magnetically performed by installing an electromagnet at the front end of the operation wire 13. Alternatively, the medical blocking tool 1 and the operation wire 13 may be connected to each other via a connection portion which can be melted by heat, and an electrical current may be supplied thereto so that the connection portion is melted by generated heat. Alternatively, a predetermined liquid may be sprayed to the medical blocking tool 1 connected to the operation wire 13 so that the medical blocking tool 1 is separated from the operation wire 13 by the pressure.

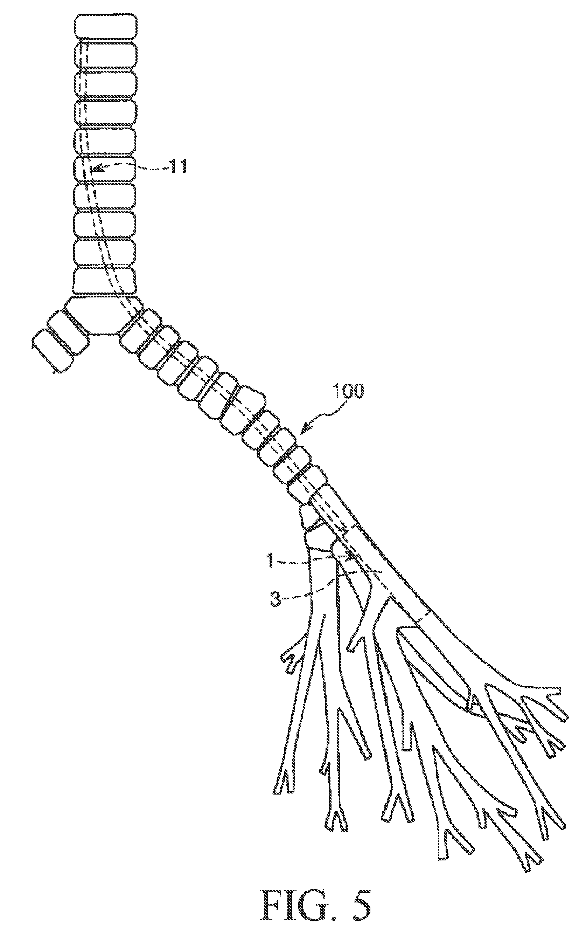

First, a bronchoscope 11 is inserted through a patient's mouth or nose, and the front end thereof is positioned near the blocking target portion of a bronchial tube 100 (refer to Fig. 5).

Next, as shown in Figs. 3 and 4, the medical blocking tool 1 is inserted into a lumen 12 of the bronchoscope 11, and the operation wire 13 is pressed to be moved toward the front end of the lumen 12. Accordingly, the medical blocking tool 1 is moved in the inside of the lumen 12 of the bronchoscope 11 toward the front end thereof, and is discharged from the front end of the lumen 12 to the blocking target portion of the bronchial tube 100.

As shown in Fig. 5, the swelling section 3 of the medical blocking tool 1 contacts the bodily fluids (tissue fluids) inside the bronchial tube 100, and the anionic group of the gel polymer forming the swelling section 3 is depronated, so that the swelling section 3 is swelled. Accordingly, the medical blocking tool 1 is fixed to the inside of the bronchial tube 100, and the blocking target portion of the bronchial tube 100 is blocked. In this case, for example, even when unevenness is found in the inner wall of the bronchial tube 100, the swelling section 3 can follow the uneven shape since the swelling section 3 is flexible. For this reason, the swelling section 3 can be reliably adhered to the inner wall of the bronchial tube 100. In addition, since the physical irritation of the swelling section 3 with respect to the inner wall of the bronchial tube 100 is small, it is possible to suppress granulation or the like.

Here, a predetermined liquid may be supplied to the medical blocking tool 1 discharged to the blocking target portion of the bronchial tube 100 by the use of a lumen (not shown) of the bronchoscope 11. Accordingly, it is possible to promptly and reliably swell the swelling section 3, and to swell the swelling section 3 at the substantially same time at all times. As the liquid supplied to the medical blocking tool 1, a liquid (for example, a buffer fluid) of which the pH is adjusted to be substantially equal to that of the bodily fluids inside the bronchial tube 100 is desirable.

Subsequently, the medical blocking tool 1 is separated from the front end of the operation wire 13. Accordingly, the medical blocking tool 1 is placed inside of the bronchial tube 100.

Then, at the time when the medical blocking tool 1 is placed to the next blocking target portion of the bronchial tube 100, the front end of the bronchoscope 11 is moved to the vicinity of the next blocking target portion of the bronchial tube 100, and the same operations as described above are performed. Likewise, it is possible to respectively place a plurality of medical blocking tools 1 to the corresponding blocking target portions while the bronchoscope 11 remains inserted into the bronchial tube 100. Accordingly, since it is possible to shorten the treatment time, it is possible to reduce the burden on the patient.

Subsequently, in the same way, a predetermined number of medical blocking tools 1 are respectively placed to the corresponding blocking target portions, and the bronchoscope 11 is removed from the bronchial tube 100.

In addition, the medical blocking tool 1 can be easily extracted even after the placement inside of the bronchial tube 100.

At the time when the medical blocking tool 1 is extracted, a predetermined liquid is supplied to the medical blocking tool 1 placed inside of the bronchial tube 100 by the use of a lumen (not shown) of the bronchoscope 11. As the liquid, a liquid (for example, a buffer fluid) of which the pH is adjusted to be smaller than that of the bodily fluids inside the bronchial tube 100 is used. Accordingly, the anionic group of the gel polymer forming the swelling section 3 is protonated, so that the swelling section 3 is contracted. Accordingly, it is possible to easily extract the medical blocking tool 1.

As described above, according to the medical blocking tool 1, it is possible to easily and promptly place the medical blocking tool 1 to the blocking target portion of the bronchial tube, and to reliably block the blocking target portion.

In addition, in the invention, a plurality of the core sections may be provided.

### illustrative example

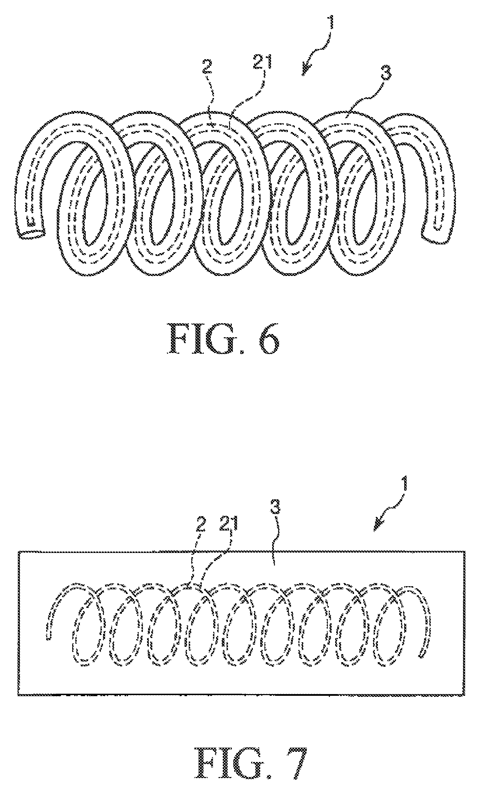

Fig. 6 is a side view showing a second illustrative example of the medical blocking tool not falling under the scope of the claims.

Hereinafter, the second illustrative example will be described. However, the differences from the first embodiment will be mainly described, and description of the similarities will be omitted.

As shown in Fig. 6, in the medical blocking tool 1 of the second illustrative example not falling under the scope of the claims, the core section 2 is formed as a coil-shaped wire rod 21.

In addition, the swelling section 3 is formed as a coil shape along the wire rod 21 of the core section 2, and coats the entire part of the wire rod 21 (core section 2). When the swelling section 3 is swelled, a gap in the swelling section 3 is removed, so that the bronchial tube is blocked.

According to the medical blocking tool 1, it is possible to obtain the same advantage as the first illustrative example not falling under the scope of the claims.

Then, in the medical blocking tool 1, the contact area between the core section 2 and the swelling section 3 is large, and hence the adhesion between the core section 2 and the swelling section 3 is improved.

### Third Embodiment

Fig. 7 is a side view showing a third illustrative example of the medical blocking tool not falling under the scope of the claims.

Hereinafter, the third illustrative example not falling under the scope of the claims will be described. However, the differences from the first illustrative example not falling under the scope of the claims will be mainly described, and description of the similarities will be omitted.

As shown in Fig. 7, in the medical blocking tool 1 of the third illustrative example not falling under the scope of the claims, the core section 2 is formed as a coil-shaped wire rod 21.

In addition, the swelling section 3 is formed in a bar shape to coat the entire part of the wire rod 21 of the core section 2.

According to the medical blocking tool 1, it is possible to obtain the same advantage as the first illustrative example not falling under the scope of the claims.

Then, in the medical blocking tool 1, the contact area between the core section 2 and the swelling section 3 is large, and hence the adhesion between the core section 2 and the swelling section 3 is improved.

### Preferred Embodiment

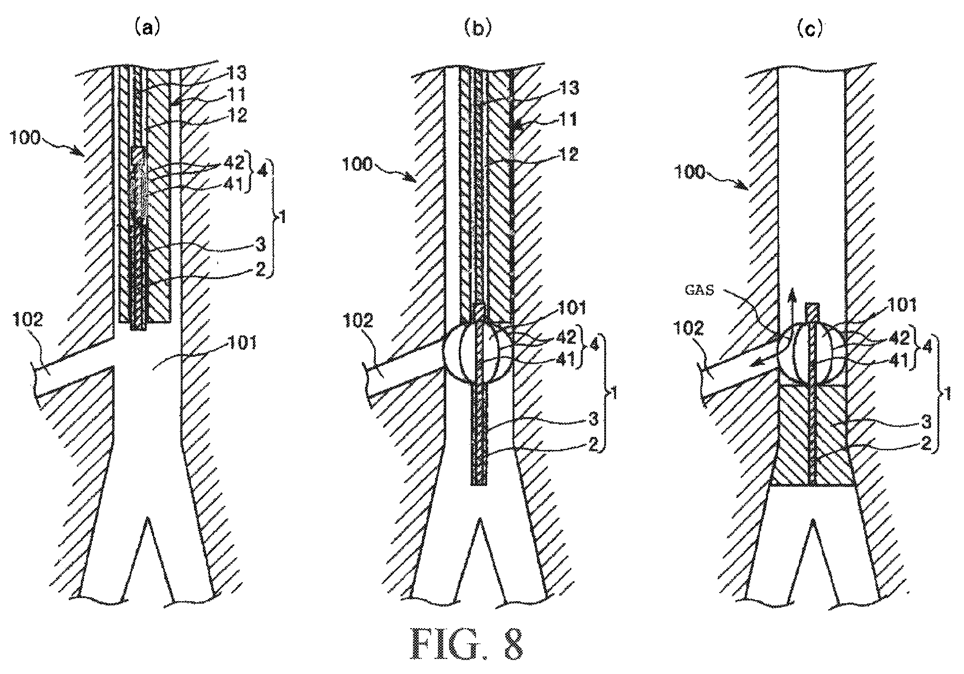

Figs. 8A, 8B, and 8C are longitudinal sectional views (diagrams illustrating instructions for use) showing a preferred embodiment of the medical blocking tool of the invention. In addition, in the following description, the downside of Figs. 8A, 8B, and 8C indicates the "front end", and the upside thereof indicates the "base end (rear end)".

Hereinafter, the preferred embodiment will be described. However, the differences from the first illustrative example not falling under the scope of the claims will be mainly described, and description of the similarities will be omitted.

This preferred embodiment is the same as the first illustrative example not falling under the scope of the claims except that a fixation assisting means is further provided.

The medical blocking tool 1 shown in Figs. 8A, 8B, and 8C further includes a fixation assisting means 4. The fixation assisting means 4 is used to assist a fixation operation of fixing the swelling section 3 to the inside of the bronchial tube 100.

The fixation assisting means 4 includes an extension portion 41 which is disposed on the side of the base end of the swelling section 3 and extends from the core section 2 to base end, and a plurality of lines of installation wires 42 which is installed at both ends of the extension portion.

The extension portion 41 may be integrally formed with the core section 2. Also, the extension portion 41 may be separately formed with the core section 2, and the separate member may be connected to the core section 2.

Each of the installation wires 42 is formed of alloy which exhibits hyperelasticity in the inside of the body. Accordingly, each of the installation wires 42 can be reliably deformed from the contracted state (refer to Fig. 8A) to the expansion state (refer to Figs. 8B and 8C), and can have an accurate restored shape in the expansion state. Here, an alloy which exhibits the hyperelasticity in the inside of the body means an alloy which has a property in which the alloy can be restored to substantially its original shape even when the alloy is deformed (bent, stretched, or compressed) up to an area where the composition of general metal is changed around at least a body temperature (around 37°C), and examples thereof include shape-memory alloy, hyperelastic alloy, and the like. The types of the shape-memory alloy and the hyperelastic alloy are not particularly limited, but for example, titanium-based alloy (Ti-Ni, Ti-Pd, Ti-Nb-Sn, and the like) or copper-based alloy is desirable. For example, desirable composition containing titanium of about 30 to 52 atom% and a remainder of nickel and one or more additional alloy component of 10 atom% or less may be exemplified.

In the medical blocking tool 1 (fixation assisting means 4) with such a configuration, when the medical blocking tool 1 is accommodated in the inside of the lumen 12 of the bronchoscope 11 as shown in the state of Fig. 8A, each of the installation wires 42 is regulated by the inner peripheral surface of the bronchoscope 11. That is, the inner peripheral surface of the bronchoscope 11 applies an external force to the corresponding installation wire 42 against elasticity of each of the installation wires 42. Accordingly, each of the installation wires 42 is contracted.

When the medical blocking tool 1 is protruded from the bronchoscope 11 as shown in Figs. 8B and 8C, each of the installation wires 42 is expanded by its elasticity since the external force applied from the inner peripheral surface of the bronchoscope 11 is released. Likewise, the fixation assisting means 4 is of a so-called "self-expansion type".

In addition, as shown in Figs. 8B and 8C, the expansion operation of the installation wire 42 is performed earlier than the operation in which the swelling section 3 is expanded to be fixed to the bronchial tube 100. That is, the fixation performed by the expanded installation wire 42 and the fixation performed by the swelled swelling section 3 are carried out at different timings having a time interval therebetween. Accordingly, the temporary fixation can be performed by the installation wire 42, and the actual fixation can be performed by the swelling section 3. Therefore, it is possible to more reliably fix the medical blocking tool 1 to the bronchial tube 100.

In addition, in the fixation assisting means 4, the adjacent installation wires 42 are spaced from each other. Accordingly, for example, as shown in Figs. 8B and 8C, when the fixation assisting means 4 of the medical blocking tool 1 is positioned to a branch portion 101 of the bronchial tube 100, a gas can flow to branched portions 102 branched from the branch portion 101 via a gap between the adjacent installation wires 42. That is, it is possible to ensure a bronchial pathway to the branched portions 102.

Further, the fixation assisting means 4 is disposed on the side of the base end of the swelling section 3 in the configuration shown in the drawings, but the invention is not limited thereto. For example, the fixation assisting means 4 may be disposed on the side of the front end of the swelling section 3.

Furthermore, the expansion/contraction portion of the fixation assisting means 4 may be formed in a coil shape.

Moreover, the fixation assisting means 4 is not limited to the self expansion type, but may be, for example, a balloon expansion type.

While the medical blocking tool of the invention has been described with reference to the embodiment shown in the drawings, the invention is not limited thereto, and the configuration of each constituent may be replaced by an arbitrary configuration having the same function. In addition, the invention may further include other arbitrary constituents or processes.

In addition, the invention may have a combination of arbitrary two or more configurations (characteristics) of the preferred embodiment with any of the illustrative examples.

Further, in the invention, the shape of the core section 2 is not limited to the above-described embodiment, but also may be formed in, for example, a particle shape (for example, a cubic shape, an elongated rectangular shape, or the like), a mesh shape (a lattice shape), or the like.

Furthermore, the medical blocking tool of the invention is not limited to the one used for blocking the bronchial tube.

The medical blocking tool of the invention is the medical blocking tool which is placed inside of the lumen of the body to block the lumen, including the core section, and the swelling section which is formed of a swelling material swelled by absorbing a liquid thereinto so that the volume thereof is increased and is provided around the core section, wherein when the swelling section is swelled, the swelling section is fixed to the inside of the lumen of the body to block the lumen. For this reason, it is possible to quickly, easily, and reliably place the medical blocking tool inside of the lumen of the body. Accordingly, the medical blocking tool of the invention has industrial applicability.

## Claims

1. A medical blocking tool (1) which is configured to be placed inside of a lumen of a body to block the lumen of the body, the medical blocking tool (1) comprising:
a core section (2); and
a swelling section (3) which is formed of a swelling material swelled by absorbing a liquid thereinto so that the volume thereof is increased and which is provided around the core section (2),
wherein when the swelling section (3) is swelled, the swelling section (3) is configured to be fixed to the inside of the lumen of the body to block the lumen of the body,
**characterized by**
a fixation assisting means (4) for assisting the fixation when the swelling section (3) is inserted into the inside of the lumen of the body, said fixation assisting means (4) including an extension portion (41) which is disposed, relative to the insertion direction, on the side of the rear end of the swelling section (3), and extends from the core section (2) in a direction opposite to said insertion direction, and a plurality of lines of installation wires (42), each of which is installed at both ends of the extension portion (41).

2. The medical blocking tool (1) according to claim 1, wherein the swelling material is a gel polymer.

3. The medical blocking tool (1) according to claim 2, wherein a part of a molecular structure of the gel polymer has an anionic group.

4. The medical blocking tool (1) according to claim 3, wherein the volume of the gel polymer is increased when contacting a liquid deprotonating the anionic group, and the volume thereof is decreased when contacting a liquid protonating the anionic group.

5. The medical blocking tool (1) according to claim 1, wherein the core section (2) is formed as a bar-shaped wire rod, and wherein the swelling section is formed along the wire rod, and coats the wire rod.

6. The medical blocking tool (1) according to claim 1, wherein the core section (2) has a contrast property.

7. The medical blocking tool (1) according to claim 1, wherein a surface of the core section (2) is subjected to a roughing process.

8. The medical blocking tool (1) according to claim 1, wherein the fixation assisting means (4) is formed of an elastic material, and
wherein the fixation assisting means (4) is configured to be contracted when applying an external force against its elasticity, and is configured to be expanded when releasing the external force.

9. The medical blocking tool (1) according to claim 1, wherein the medical blocking tool (1) is configured to be used to block a bronchial tube.

## Patentansprüche

1. Medizinisches Verschlusswerkzeug (1), das dafür konfiguriert ist, innerhalb eines Lumens eines Körpers angeordnet zu werden, um das Lumen des Körpers zu verschließen, wobei das medizinische Verschlusswerkzeug (1) Folgendes umfasst:
eine Kernsektion (2) und
eine Quellsektion (3), die aus einem Quellmaterial geformt ist, das durch das Absorbieren einer Flüssigkeit in demselben aufgequollen wird, so dass das Volumen derselben gesteigert wird, und die um die Kernsektion (2) bereitgestellt wird,
wobei, wenn die Quellsektion (3) aufgequollen wird, die Quellsektion (3) dafür konfiguriert ist, an der Innenseite des Lumens des Körpers befestigt zu sein, um das Lumen des Körpers zu verschließen,
**gekennzeichnet durch**
ein Befestigungsunterstützungsmittel (4), um die Befestigung zu unterstützen, wenn die Quellsektion (3) in die Innenseite des Lumens des Körpers eingesetzt wird, wobei das Befestigungsunterstützungsmittel (4) einen Ausdehnungsabschnitt (41), der, im Verhältnis zu der Einsetzungsrichtung, auf der Seite des hinteren Endes der Quellsektion (3) angeordnet ist und sich von der Kernsektion (2) aus in einer Richtung, entgegengesetzt zu der Einsetzungsrichtung, erstreckt, und mehrere Linien von Installationsdrähten (42), deren jede an beiden Enden des Ausdehnungsabschnitts (41) installiert ist, einschließt.

2. Medizinisches Verschlusswerkzeug (1) nach Anspruch 1, wobei das Quellmaterial ein Gelpolymer ist.

3. Medizinisches Verschlusswerkzeug (1) nach Anspruch 2, wobei ein Teil einer molekularen Struktur des Gelpolymers eine anionische Gruppe aufweist.

4. Medizinisches Verschlusswerkzeug (1) nach Anspruch 3, wobei das Volumen des Gelpolymers gesteigert wird, wenn es eine Flüssigkeit, welche die anionische Gruppe deprotoniert, berührt, und das Volumen desselben vermindert wird, wenn es eine Flüssigkeit, welche die anionische Gruppe protoniert, berührt.

5. Medizinisches Verschlusswerkzeug (1) nach Anspruch 1, wobei die Kernsektion (2) als ein stabförmiger Draht geformt ist und wobei die Quellsektion entlang des Drahtstabs geformt ist und den Drahtstab überzieht.

6. Medizinisches Verschlusswerkzeug (1) nach Anspruch 1, wobei die Kernsektion (2) eine Kontrasteigenschaft hat.

7. Medizinisches Verschlusswerkzeug (1) nach Anspruch 1, wobei die Oberfläche der Kernsektion (2) einem Aufrauvorgang unterworfen ist.

8. Medizinisches Verschlusswerkzeug (1) nach Anspruch 1, wobei das Befestigungsunterstützungsmittel (4) aus einem elastischen Material geformt ist und wobei das Befestigungsunterstützungsmittel (4) dafür konfiguriert ist, zusammengezogen zu werden, wenn eine äußere Kraft gegen seine Elastizität ausgeübt wird, und dafür konfiguriert ist, ausgedehnt zu werden, wenn die äußere Kraft gelöst wird.

9. Medizinisches Verschlusswerkzeug (1) nach Anspruch 1, wobei das medizinische Verschlusswerkzeug (1) dafür konfiguriert ist, dazu verwendet zu werden, einen Luftröhrenast zu verschließen.

## Revendications

1. Outil de blocage médical (1) qui est conçu pour être placé à l'intérieur de la lumière d'un corps afin de bloquer la lumière du corps, l'outil de blocage médical (1) comprenant :
une section centrale (2) ; et
une section de gonflement (3) qui est formée d'un matériau gonflant, gonflé par absorption d'un liquide qui y est contenu de telle sorte que son volume augmente et qui est placée autour de la section centrale (2),
dans lequel, lorsque la section de gonflement (3) est gonflée, la section de gonflement (3) est conçue pour être fixée à l'intérieur de la lumière du corps afin de bloquer la lumière du corps,
**caractérisé par**
un moyen (4) facilitant la fixation pour aider à la fixation lorsque la section de gonflement (3) est insérée à l'intérieur de la lumière du corps, ledit moyen (4) facilitant la fixation comprenant une partie d'extension (41) qui est disposée, par rapport à la direction d'insertion, sur la face de l'extrémité arrière de la section de gonflement (3), et s'étend à partir de la section centrale (2) dans une direction opposée à ladite direction d'insertion, et une pluralité de lignes de fils d'installation (42), chacun d'eux étant installé au niveau des deux extrémités de la partie d'extension (41).

2. Outil de blocage médical (1) selon la revendication 1, dans lequel le matériau gonflant est un gel polymère.

3. Outil de blocage médical (1) selon la revendication 2, dans lequel une partie d'une structure moléculaire du gel polymère a un groupe anionique.

4. Outil de blocage médical (1) selon la revendication 3, dans lequel le volume du gel polymère augmente en venant en contact avec un liquide déprotonant le groupe anionique, et son volume diminue en venant en contact avec un liquide protonant le groupe anionique.

5. Outil de blocage médical (1) selon la revendication 1, dans lequel la section centrale (2) est formée comme une tige de fil en forme de barre, et
dans lequel la section de gonflement est formée le long de la tige de fil, et recouvre la tige de fil.

6. Outil de blocage médical (1) selon la revendication 1, dans lequel la section centrale (2) a une propriété de contraste.

7. Outil de blocage médical (1) selon la revendication 1, dans lequel une surface de la section centrale (2) est soumise à une opération d'ébauchage.

8. Outil de blocage médical (1) selon la revendication 1, dans lequel le moyen (4) facilitant la fixation est fait d'un matériau élastique, et
dans lequel le moyen (4) facilitant la fixation est conçu pour se contracter lors de l'application d'une force externe à l'encontre de son élasticité, et est conçu pour se déployer lors du relâchement de la force externe.

9. Outil de blocage médical (1) selon la revendication 1, dans lequel l'outil de blocage médical (1) est conçu pour être utilisé afin de bloquer une bronche.
